# EUROPEAN PATENT APPLICATION

(11) **EP 1 167 517 A1**
(43) Date of publication of application: **02.01.2002**
(21) Application number: 00202190.5
(22) Date of filing: 22.06.2000
(51) Int. Cl.: C12N 5/00

(54) **Tissue engineering**

(71) Applicant: IsoTis N.V., 3723 MB Bilthoven (NL)
(72) Inventor: de Bruijn, Joost, Dick, 3817 JL Amersfoort (NL); van Apeldoorn, Aart, Alexander, 3564 BS Utrecht (NL); Peters, Wilhelmina, Hendrika, Lamberta, 3706 AM Zeist (NL)
(74) Representative: Prins, Adrianus Willem

(57) **Abstract**

The invention relates to a specific process for producing tissue *in vitro.* The present approach to tissue engineering allows for an optimal control of the culturing process, leading to proliferation or differentiation as desired, and its conditions. Particularly, the invention facilitates the maintenance of sterile conditions.

## Description

The invention relates to the field of tissue engineering. More specifically, the invention relates to a process for the *in vitro* culturing of cells to produce tissue of a predetermined type.

Over the past two decades, extensive research efforts have been committed to the field of tissue engineering. Numerous problems in the field of substitution medicine triggered these efforts. Implants of various types, be it bone implants or organ transplants, that are used in current medical practice suffer from the great disadvantage that they are of materials foreign to the receiving patient's body.

For instance, hip prosthesis are often manufactured of metals. In order to reduce rejection reactions in the patient and to enhance attachment of the patient's cells, several complicated precautions are needed. One such, widely taken precaution is the provision of a calcium phosphate coating on the metal implant. Upon transplantation of organs obtained from a donor, such as kidneys or livers, extreme care must be taken to avoid rejection of the transplant due to strong reactions of the immune system of the receiving patient. Even though donors are found through a very thorough selection process, strong immune suppressants are always necessary.

Tissue engineering provides a more elegant approach to substitution medicine. Principally, it makes use of cells of the patient himself. These cells are taken from the patient's body in a biopsy, after which they are cultured *in vitro* to produce the tissue that the patient requires. For instance, a bone marrow biopsy might be taken from a patient in need of a hip prosthesis. The bone marrow cells might then be seeded onto a suitable scaffold. This is often a three dimensional structure of a biocompatible, biodegradable material. After seeding, the bone marrow cells are allowed to grow and proliferate. Depending on the circumstances, they may also be allowed to differentiate to provide cells of a certain predetermined type. Once the objective tissue has developed to a sufficient extent, the prosthesis may be implanted. The scaffold provides much of the coherency and mechanical strength of the prosthesis in the first stages after implantation. Eventually, the scaffold may degrade *in vivo* and be replaced by new bone tissue. As this approach involves the use of autologous cells only, problems due to immune responses are avoided entirely.

When producing medical implants by way of tissue engineering, it is of great importance to maintain sterile conditions. It will not need an elaborated discussion to illustrate the disadvantages and the dangers of microbial infections of the *in vitro* produced autologous tissue before implantation. Complete avoidance of microbial infections is, however, difficult to attain.

Possibly partly due to the fact that the field of tissue engineering is still relatively young, no practical solution to this problem has yet been proposed in the art.

The present invention seeks to provide a practical and feasible approach to tissue engineering which obviates at least partially most, if not all, of the problems of maintaining sterile conditions throughout the tissue engineering process. The objective approach should furthermore enable a suitable upscaling of the process to allow for an efficient and economical scheme for carrying out the production of several tissue samples, optionally of different types, for different patients in a parallel manner.

In accordance with the invention, there is provided a process for *in vitro* producing tissue, wherein cells are cultured in a vessel provided with an inlet and an outlet for culture medium, which vessel is perfused with culture medium.

A process according to the invention provides many convenient ways of manipulating the tissue production without affecting the sterile conditions within the vessel. In the context of the invention, when something is kept under 'sterile conditions' is intended to indicate that it is substantially prevented from exposure to microorganisms, such as viruses, bacteria and fungi. Furthermore, it has been found that the dynamic conditions that arise from the perfusion of the culture medium lead to a more homogeneous distribution and growth of the cells over the scaffold.

Moreover, the perfusion of the culture medium results in optimal culturing conditions in the vessel. In addition, the invention allows for the possibility of obtaining large cell amounts in a small volume with a large surface. Further, the addition of certain additives to the culture medium can be manipulated quickly and efficiently, because of the continuous medium flow around the cells. Moreover, many of the relevant parameters of the tissue engineering process, such as pH, oxygen level, the growth of the cells and the like, can be monitored in an optimal manner. The vessel that is used for the culturing can advantageously be used as a transport container, enabling transport of the engineered product substantially without affecting sterility.

For culturing of patient derived cells on scaffolds, the use of separate small bioreactors is preferred. It is further preferred that for each patient a separate bioreactor is used. In this way it is possible to avoid cross contamination between patients. It is also possible that more than one scaffold, intended for the same patient, are present in one bioreactor. Instead of culturing cells on separate scaffolds, culturing on a large bulk of scaffold at the same time means a reduction of the amount of procedures one needs to perform per patient. It is also advantageous that culturing for different patients may be performed under substantially the same conditions to allow a standardized process regarding quality. In a bioreactor, various crucial cell culture parameters can be registered, monitored and controlled. Adjustment of these parameters allows for creating a perfect cell growth environment.

It is preferred to use a bioreactor that can be closed easily before transportation to the hospital and is configured to hold a large bulk of cell culture scaffold, on which cells can easily be cultured. In this way the bioreactor does not have to be opened before transportation. Also, the sample does not have to be put into another tube, thus preventing the product from getting infected. Therefore, a bioreactor of which, all tubes can be cut off easily without causing leakage of medium is preferred.

The tissue that is produced according to the invention may in principle be of any type. Preferably, the tissue will be human and may be one of bone, cartilage, tendon, ligament, muscle, skin, gland or organ tissue. Generally, the cells that are cultured to produce the objective tissue will have been obtained from a biopsy from a patient for which the tissue is intended. These cells may be of any type that is capable of proliferating and differentiating to produce the objective tissue. Typical examples of cell types that may be employed include fibroblasts, chondrocytes, keratinocytes, muscle cells (myocytes), satellite cells, (bone) marrow cells, mesenchymal stem cells, mesenchymal stem cells, melanocytes, mesothelial cells, bone forming cells, hepatocytes, intestinal cells, kidney cells, fat tissue forming cells, hair follicle forming cells, blood vessel cells, nerve cells, and schwann cells.

The cells may be brought in the vessel directly after taking the biopsy. Alternatively, the cells may be cultured on normal tissue culture plastic prior to introduction into the vessel, in order to obtain a sufficient amount of cells.

Preferably, they are seeded onto a scaffold that is already present in the vessel. The scaffold may be of metallic, ceramic or polymeric material, or of a combination of two or more of said materials. For instance, a polymeric scaffold provided with a ceramic coating may be utilized. Preferably, the scaffold is of biodegradable and biocompatible material. In the context of the present application, the term biocompatible is intended to refer to materials which may be incorporated into a human or animal body substantially without unacceptable responses of the human or animal. The term biodegradable refers to materials which, after a certain period of time, are broken down in a biological environment. In certain preferred embodiments, the rate of breakdown is chosen similar or close to the rate at which the body generates autogenous tissue to replace the implant of which the biodegradable material is manufactured.

The scaffold preferably provides the desired shape of a medical implant. For skin tissue it may for example have the form of a sheet. For other applications, its structure will be more complicated and three dimensional. Furthermore, the scaffold may, depending on the objective tissue and the conditions under which the tissue is produced, be porous, dense, fibrous or a combination thereof.

In order to culture the cells and to convert them into the desired tissue, use is made of a culture medium. It is an important aspect of the invention that the culture medium is refreshed constantly during the culturing process. Accordingly, its composition may be adjusted to the transitory phases of the tissue producing process. Preferably, the culture medium is oxygenated prior to entry of the vessel to allow for optimum conditions for the culturing process.

The composition of the culture medium will be chosen dependent on the nature of the cells that are being cultured, the nature of the objective tissue and the transitory phase of the tissue producing process. The choice for the exact composition can be optimized by the skilled person, based on his general knowledge of tissue engineering technology. Examples of suitable culture media, which may be used as such or adjusted to the circumstances, are Dulbecco's Modified Eagle Medium (DMEM), alpha Minimal Essential Medium (α-MEM), HAM-F12 (originally developed by Ham for CHO cells), McCoy's medium (formulated by McCoy in 1959) and RPMI (developed by Moore et al. at the Roswell Park Emorial Institute).

As said before, the culture medium is refreshed constantly during the culturing process. The rate (flow) of the perfusion can conveniently be adapted to the other parameters of the process by the skilled person, based on his normal knowledge of the art. The culture medium that has left the vessel may either be adjusted in composition and be reused, or it may be discarded, necessitating the constant availability of fresh culture medium.

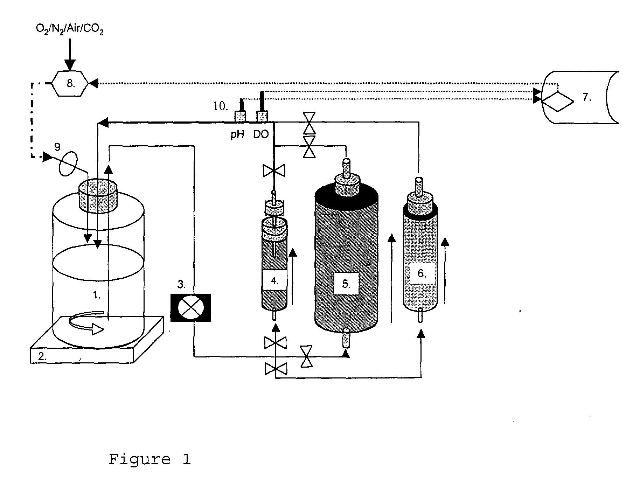

The present process will now be further illustrate with reference to the attached Figure 1. It will be clear that the invention is not restricted to the choices depicted in said figure, but that many variations and alterations are possible without leaving the scope of the invention.

The system shown in Figure 1 consists basically of one or several cell adherent material containing tubes linked by a peristaltic pump (3) to a culture medium-containing vessel (1), preferably provided with a stirrer (2). The medium is pushed through the tubes and can in this way provide for a suitable cell growth environment.

Adherent cells are first cultured on a scaffold to proliferate the cells to a certain required amount of cells. For cell culture expansion a large surface area in a small volume is important. This first stage is preferably carried out in a first stage cell culture cartridge (4).

In sequential cartridges the proliferation will take place (5) and subsequently in a last cartridge (6) tissue engineering of a scaffold will be done, it is however also possible to combine proliferation and tissue engineering in one cartridge. Optionally, the culture expansion phase can be performed in normal tissue culture flasks, e.g. in case no suitable scaffold might be available for a specific cell type.

The cartridges are preferably linked by silicone tubing, which can be closed by a valve (11), to ensure a closed sterile system.

After first stage proliferation in a relative small cartridge (4) the cells are at one point (determination maybe by measuring O₂ uptake) trypsinized and pumped into a larger cartridge (5), were the cells can again be proliferated in a larger volume.

The pH of the medium can be detected by pH sensors (10), which can also be monitored and regulated by addition of CO₂ by automated gas valves (8). In order to ensure the maintaining of sterile conditions, the gases are preferably introduced into the vessel (1) via a sterile gas filter inlet (9).

Cell growth can be followed based on the OUR (Oxygen Uptake Rate), which can be calculated from the control output of a DO-controller (Dissolved Oxygen). The oxygen concentration is preferably kept at a constant level with the gasses (8) controlled by the controlling unit (7). This is important when cells start to proliferate and the cell density is increasing.

After proliferation the cells can again be trypsinized in the cartridge and then be pumped into the last cartridge, in which a material is placed which, together with the cells, will eventually produce the end product.

Several important cell-culture parameters like oxygen uptake, pH, temperature, etc., can be monitored and regulated by a series of sensors (10) and a biocontroller unit (7) during the culture period.

A closed cell culture system can provide for a solution regarding sterility, easy handling and standardized cell culture. The aim is to culture cells on a scaffold placed in a tube, through which cell culture medium is circulating. The device shown in Figure 1 provides for the possibility to culture a large amount of cells on a material, with a high surface area, in a relatively small volume. The idea is to culture cells on increasing surfaces in small volumes in sequence; in order to culture expand cells. After culture expansion the cells can easily be harvested from the tubes to be brought into a tube containing a scaffold for tissue engineering.

The invention will now be further elucidated by the following, non-restrictive example.

### EXAMPLE: Culturing Human Bone Marrow Cells on hydroxyapatite in a closed circulation system.

Porous CaP or hydroxyapatite particles of size 2 by 3.5 mm are used as a bone filler for culturing the released HBMC's on. In short harvested HBMC's are seeded in a density of 100.000-200.000 cells/particle into the particles-containing cartridge and are let to attach. Approximately 25-50 E 6 cells are needed to seed an amount of 10 cc of porous particles (40 cc corresponds to about 1600 particles having a diameter of 3.35 mm), indicating the difficulty of culturing cells on large amounts (40 cc) of porous particles. The cartridge is then again aseptically linked to a medium stock vessel and a peristaltic pump. The cells are cultured during one week using α-MEM containing 10%FBS, 0.2mM AsAP, 10 nM Dexamethason (Dex) and 10mM beta glycerophosphate (βGP) before implantation to ensure extracellular matrix formation. The cartridge ensures sterile transportation to the clinic and facilitates easy handling of a large amount of tissue engineered bone filler.

### Material and methods

Human Bone Marrow Cells obtained from a biopsy of a iliac crest of a 17-year-old female patient were seeded into tissue culture flasks at a density of 5E5 cells/cm² in α-MEM containing 10% FBS, 0.2 mM ASAP and 100U/ml penicillin and 100 µg/ml streptomycin (pen/strep) and 1% (50U/ml) heparin (added in primary cultures only). Before seeding the cells on hydroxyapatite they were cultured on normal tissue culture plastic in culture medium consisting of α-MEM containing 15% FBS, 0.2 mM ASAP and pen/strep, until 80% confluency. The cells were detached by washing the cells with warm PBS (± 37°C) and incubation for 15 min. with 0.25% Trypsin-EDTA The cells were then thoroughly resuspended and counted and reseeded in tissue culture flasks at a density of 5000 cells/cm². In this way, cells were cultured until an amount of 100 E 6 cells of the third passage was obtained to seed on 20 cc of porous hydroxyapatite. After trypsinisation, before seeding, the cells were centrifuged at 300 g for 10 min at 4°C. Subsequently the supernatant was discarded and the cells were resuspended in 5 ml of culture medium. The cell suspension was then seeded into the cartridge containing 20 cc of porous hydroxyapatite and then totally filled with culture medium. After 4-6 hours of cell attachment the cartridge is connected to a stock vessel containing 300 ml of α-MEM containing 10% FBS, 0.2 mM ASAP, 10 nM Dexamethason (Dex) and 10mM beta glycerophosphate (βGP) and pen/strep by silicone tubing. The medium is then recycled through the cartridge by a peristaltic tubing pump at low flow rate. The cells are then cultured in this system for 7 days, while continuously pumping. After 7 days the tissue-engineered hydroxyapatite is fixated in 0.14 M cacodylate buffer/3.5% glutaraldehyde for at least 15 min., subsequently the samples were stained by methylene blue staining to check morphology. A few samples were also fixated in para formaldehyde for standard immunological staining. Control samples were cultured statically in a bacteriological graded plate, by placing a droplet of cell suspension containing 2E5 cells directly on a hydroxyapatite sample. After incubation for four hours 2 ml of culture medium was supplied to the particles, which was refreshed 3 times during 7 days of culturing.

### Results

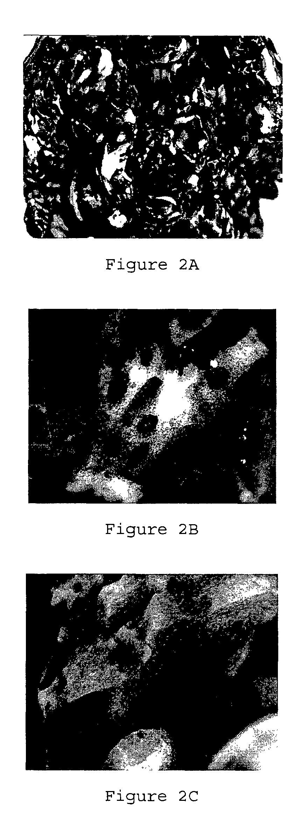

Human bone marrow cells of the third passage cultured for 7 days in a perfusion system (pictures taken by stereomicroscopy). The pictures are shown in Figures 2A, 2B and 2C.

Observation by stereomicroscopy revealed a uniform cell layer (dark blue coloration) around the porous hydroxyapatite (white coloration), which could be observed throughout the scaffold-containing cartridge. A more detailed close up of the porous samples revealed that cells also grow into the pores of the scaffold material. Single control samples (particles) seeded statically with 2E5 cells and cultured individually in a bacteriological graded plate showed a less homogeneous appearance, indicating that there was a difference regarding tissue engineering observed after 7 days.

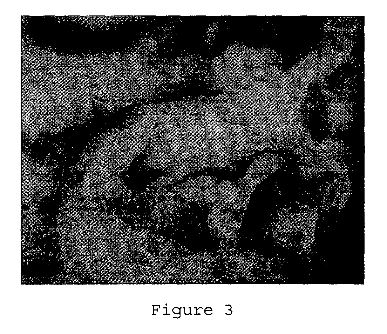

Human bone marrow cells of the third passage cultured for 7 days, in a perfusion system were also stained by a collagen type I immunological staining with peroxidase are shown in Figure 3. Positive staining indicating the presence of collagen type I is revealed by a red-brown coloration due to peroxidase activity. The distribution of collagen type I positive cells and structures showed that a considerable amount of collagen was formed after 7 days of tissue-engineering, indicating extracellular matrix formation.

The above results indicate that culturing cells on large amounts of bone filler particles according to the method described above is feasible in this manner.

## Claims

1. A process for *in vitro* producing tissue, wherein cells are cultured in a vessel provided with an inlet and an outlet for culture medium, which vessel is perfused with culture medium.

2. A process according to claim 1, wherein the culture medium is oxygenated prior to entering the vessel.

3. A process according to claim 1 or 2, wherein the culture medium is varied in composition to induce proliferation or differentiation of the cells.

4. A process according to any of the preceding claims, wherein the vessel is provided with control means for controlling the flow and composition of the culture medium and the conditions in the vessel.

5. A process according to any of the preceding claims, wherein the culture medium is recycled.

6. A process according to any of the preceding claims, wherein the tissue is human tissue.

7. A process according to claim 6, wherein the tissue is bone, cartilage, tendon, ligament, muscle, skin, gland or organ tissue.

8. A process according to any of the preceding claims, wherein the cells are cultured on a scaffold.

9. A process according to claim 8, wherein the scaffold is of metallic, ceramic or polymeric material, or of a combination of two or more of said materials.

10. A process according to any of the preceding claims, wherein the tissue is transported to a site of surgery in said vessel.

11. A process according to any of the preceding claims, wherein the cells and the tissue are kept under sterile conditions.
